Europäisches Patentamt
European Patent Office
Office européen des brevets

(19)

(11) Publication number: **0 378 285 A1**

(12) **EUROPEAN PATENT APPLICATION**

(21) Application number: **90250006.5**

(22) Date of filing: **05.01.90**

(51) Int. Cl.5: **C07C 43/29, A01N 31/08, A01N 31/04, A01N 33/08, A01N 43/02, A01N 43/40, C07C 43/285, C07C 43/295, C07C 43/164, C07C 49/753, C07C 217/76**

(30) Priority: **06.01.89 DE 3900541**

(43) Date of publication of application: **18.07.90 Bulletin 90/29**

(84) Designated Contracting States: **AT BE CH DE DK ES FR GB GR IT LI LU NL SE**


(71) Applicant: **SCHERING AKTIENGESELLSCHAFT**
**Müllerstrasse 170/78**
**D-1000 Berlin 65(DE)**

(72) Inventor: **Franke, Heinrich, Dr.**
**Fabeckstrasse 38**
**D-1000 Berlin 33(DE)**
Inventor: **Köhn, Armin, Dr.**
**Alt-Wittenau 63c**
**D-1000 Berlin 26(DE)**
Inventor: **Joppien, Hartmut, Dr.**
**Juttastrasse 18**
**D-1000 Berlin 37(DE)**


(54) **Substituted alkenes, processes for their preparation and their use as pesticides.**


(57) There are described new substituted alkenes of of general formula I

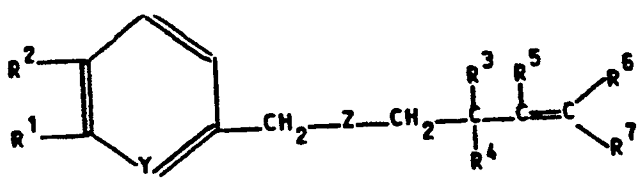

(I),

in which in which $R^1$, $R^2$, $R^3$, $R^4$, $R^5$, $R^6$, $R^7$, Y and Z, have the meanings given in the description and processes for their preparation. The compounds can be used as pesticides, especially against insects and mites.


EP 0 378 285 A1

**Substituted alkenes, processes for their preparation and their use as pesticides.**

The invention relates to new substituted alkenes, their preparation and their use as pesticides especially against insects and mites.

It is known that certain substituted alkenes (EP 227 369) and allyl substituted benzyl ethers (DE 2 709 355) have insecticidal properties.

The disadvantage of the known compounds however is that the insecticidal and acaricidal activity not sufficiently high.

The object of the present invention is to provide new compounds that combat insects and mites better than compounds known for this purpose.

It has now been found that substituted alkenes of of general formula I

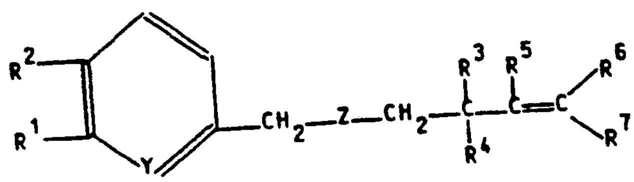

(I),

in which

$R^1$ is phenyl, phenoxy or anilino, which can be substituted by halogen, $C_{1-4}$-alkyl or $C_{1-4}$-alkoxy;

$R^2$ is hydrogen or fluorine;

$R^2$ and $R^4$, independently of each other, are $C_{1-4}$-alkyl or together form a 3-6 membered ring;

$R^5$ is $C_{1-6}$-alkyl, $C_{1-6}$-haloalkyl, phenyl or halogen;

$R^6$ is hydrogen, halogen, $C_{1-6}$-alkyl, $C_{1-6}$-haloalkyl, $C_{2-6}$-alkenyl or phenyl; or

$R^5$ and $R^6$ together form a ring, which can be substituted by halogen or alkyl and can contain a further double bond and/or an O, N or S atom;

$R^7$ is hydrogen, halogen or $C_{1-4}$-alkyl

Y is CH, $CCH_3$ or N; and

Z is O, S or $CH_2$,

show better insecticidal and acaricidal activity in comparison with known compounds.

Some of the compounds of the invention exist as optical or geometric isomers. The invention includes all isomers as well as their mixtures.

The compounds of the invention of general formula I of the invention can be prepared by

a) reacting a compound of general formula II

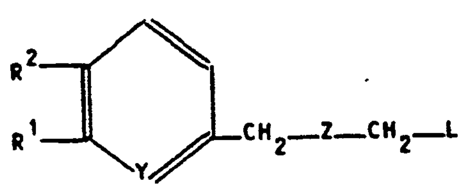

(II),

in which L is Cl, Br mesylate or tosylate, with an anion of a carboxylic acid ester of general formula III

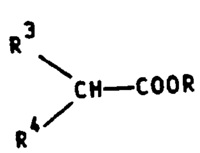

(III)

in which R is methyl or ethyl, then reacting with an excess of a Grignard reagent of formula $R'MgHal$, in which $R'$ is $C_{1-6}$-alkyl and Hal is Cl, Br or I, and dehydrating the resulting tertiary alcohol with a dehydrating reagent; or

b) when Z in formula I is O or S, reacting a dianion of a carboxylic acid of general formula IV

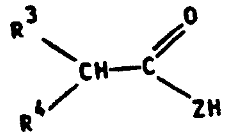

(IV)

with a ketone of general formula V,

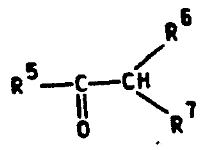

(V)

esterifying the resulting 2-hydroxyacid with simultaneous removal of water, reducing the ester with lithium aluminium hydride to give the compound of formula VI

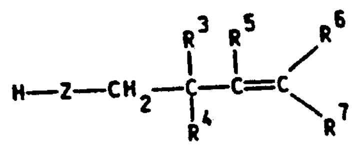

(VI)

and then etherifying this compound in the presence of a base with a compound of formula VII;

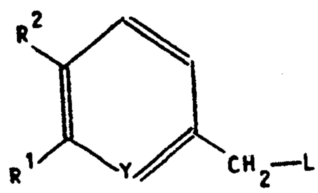

(VII)

or

c) reacting a ketone of general formula VIII

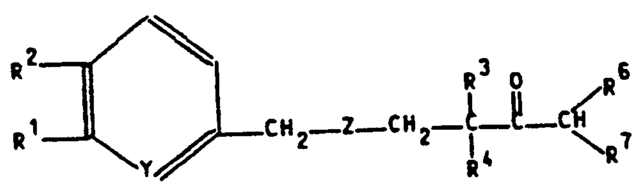

(VIII),

with a Grignard reagent of formula R′MgHal, and dehydrating the resulting tertiary alcohol with a dehydrating reagent; or

d) when $R^5$ in formula I is halogen, reacting a ketone of general formula VIII, with a halogenating reagent, with removal of water, in which $R^1$, $R^2$, $R^3$, $R^4$, $R^5$, $R^6$, $R^7$, Y and Z, have the meanings given in formula I.

The preparation and reaction of the anions and dianions can be carried out in known manner, such as described in eg Synthesis 1982, 521 ff.

In process d), phosphorus pentachloride is especially suitable as the halogenating agent.

The purification of the end product is carried out as a general rule by column chromatography.

The compounds of the invention are, as a rule, almost colourless and odourless liquids that are almost insoluble in water, yet highly soluble in aliphatic hydrocarbons, such as hexane and halogenated hydrocarbons, such as chloroform, methylene dichloride and carbon tetrachloride, aromatic hydrocarbons such as benzene, toluene and xylene, ethers, such as diethyl ether, tetrahydrofuran and dioxane, nitriles, such as

acetonitrile, alcohols, such as methanol and ethanol, amides, such as dimethylformamide, and sulphoxides, such as dimethyl sulphoxide.

The phenyl or pyridinyl derivatives of general formula II, used as starting materials, are either known or can be prepared in known manner. The carboxylic acids of formula IV and ketones of formula V are known compounds.

The compounds of the invention are distinguished by good insecticidal and acaricidal activity and thus represent a valuable improvement in the state of the art. Based on their activity against a wide range of sucking arthropods, the compounds of the invention can be used not only against pests in crops but also for combating human and domestic animal parasites. In plants where the pests cannot be directly treated, prophylactic treatment of fodder plants is sufficient. The activity of the compounds of the invention is of particular importance against parasites which have developed resistance to other substances.

Examples of insects, including animal ectoparasites, that can be combated by the compounds of the invention include Lepidoptera, such as Plutella xylostella, Spodoptera littoralis, Heliothis armigera and Pieris brassicae; Diptera, such as Musca domestica, Ceratitis capitata, Erioischia brassicae, Lucilia sericata and Aedes aegypti; Homoptera, including aphids such as Megoura viciae and Nilaparvata lugens; Coleoptera, such as Phaedon cochleariae, Anthonomus grandis and corn rootworms (Diabrotica spp. eg. Diabrotica undecimpunctata); Orthoptera, such as Blattella germanica; ticks, such as Boophilus microplus and lice, such as Damalinia bovis and Linognathus vituli, as well as mites such as Tetranychus urticae and Panonychus ulmi.

The compounds according to the invention can be used at a concentration of 0.0005 to 5%, preferably from 0.001 to 1%, calculated as gram active material per 100 ml of the composition.

The compounds of the invention can be used either alone or in mixture with each other or another insecticide.

Optionally other plant protection or pesticidal compositions, such as for example insecticides, acaricides or fungicides can be added depending on the desired result.

An improvement in the intensity and speed of action can be obtained, for example, by addition of suitable adjuvants, such as organic solvents, wetting agents and oils. Such additives may allow a decrease in the dose.

Suitable mixture partners may also include phospholipids, e.g. such as from the group phosphatidylcholine, hydrogenated phosphatidylcholine, phosphatidylethanolamine, N-acyl-phosphatidylethanolamine, phosphatidylinositol, phosphatidylserine, lysolecithin or phosphatidylglycerol.

The designated active ingredients or their mixtures can suitably be used, for example, as powders, dusts, granules, solutions, emulsions or suspensions, with the addition of liquid and/or solid carriers and/or diluents and, optionally, binding, wetting, emulsifying and/or dispersing adjuvants.

Suitable liquid carriers are, for example aliphatic and aromatic hydrocarbons such as benzene, toluene, xylene, cyclohexanone, isophorone, dimethyl sulphoxide, dimethylformamide, other mineral-oil fractions and plant oils.

Suitable solid carriers include mineral earths, e.g. tonsil, silica gel, talcum, kaolin, attapulgite, limestone, silicic acid and plant products, e.g. flours.

As surface-active agents there can be used for example calcium lignosulphonate, polyoxyethylenealkylphenyl ether, naphthalenesulphonic acids and their salts, phenolsulphonic acids and their salts, formaldehyde condensates, fatty alcohol sulphates, as well as substituted benzenesulphonic acids and their salts.

The percentage of the active ingredient(s) in the various preparations can vary within wide limits. For example, the compositions can contain about 10 to 90 percent by weight active ingredients, and about 90 to 10 percent by weight liquid or solid carriers, as well as, optionally up to 20 percent by weight of surfactant.

The agents can be applied in customary fashion, for example with water as the carrier in spray mixture volumes of approximately 100 to 3,000 l/ha. The agents can be applied using low-volume or ultra-low-volume techniques or in the form of so-called microgranules.

Formulations can be prepared, for example, from the following ingredients.

A WETTABLE POWDER

20 percent by weight active ingredient
35 percent by weight bentonite
8 percent by weight calcium lignosulphonate
2 percent by weight of the sodium salt of N-methyl-N-oleyltaurine
35 percent by weight silicic acid

B PASTE

45 percent by weight active ingredient
5 percent by weight sodium aluminium silicate
15 percent by weight cetylpolyglycol ether with 8 moles ethylene oxide
2 percent by weight spindle oil
10 percent by weight polyethylene glycol
23 parts water

C EMULSIFIABLE CONCENTRATE

20 percent by weight active ingredient
75 percent by weight isophorone
5 percent by weight of a mixture based on the sodium salt of N-methyl-N-oleyltaurine and calcium lignosulphonate

The following Examples illustrate the preparation of compounds of the invention.

Example 1

6-(3-Phenoxyphenyl)-2,3,3,-trimethyl-1-hexene

1. 2.06 g (17.7 mMol) ethyl isobutyrate, dissolved in 5 ml tetrahydrofuran (THF), was added dropwise at -60° C, to a solution of 20.0 mMol lithium diisopropylamide (prepared from diisopropylamine and n-butyllithium at 0° C) in 10 ml THF. The mixture was stirred for 45 minutes and in succession there was added, dropwise, 5.0 g (17.2 mMol) 3-(3-phenoxyphenyl)propyl bromide in 10 ml THF and 1.3 ml hexamethylphosphorotriamide (HMPT), at -60° C. The mixture was warmed to room temperature, stirred for 30 minutes and poured into ice-water. After addition of aqueous ammonium chloride, the mixture was shaken with ether and the organic phase dried and evaporated. After chromatography on silica gel, there remained 4.0 g (71% of theory) of ethyl 2,2-dimethyl-5-(3-phenoxyphenyl)valerate.
$n^{20}_{D}$: 1.5270

2. A Grignard reagent solution was prepared from 2.4 g (0.1 Mol) of magnesium turnings and 8.1 ml (0.13 mol) methyl iodide in 60 ml ether. To this solution was added dropwise, 30.2 g (40.4 mMol) of the ester prepared under 1, dissolved in 30 ml ether, and the mixture heated under reflux for 2 hours. It was then poured into water, aqueous ammonium chloride added, and the mixture shaken with ether, the organic phase dried with magnesium sulphate and evaporated. Chromatography on silica gel gave 5.75 g (46.% of theory) of 6-(3-phenoxyphenyl)-2,3,3-trimethylhexane-2-ol.
$n^{20}_{D}$: 1.5491

2.5 g (8 mMol) of the tertiary alcohol prepared from 2 was dissolved in 5 ml pyridine and treated under ice cooling with 2.5 ml phosphorus oxychloride. The mixture was stirred for 4 hours at room temperature, poured into ice water, acidified with aqueous hydrochloric acid and extracted with ether. After washing twice with water, the organic phase was dried with magnesium sulphate and evaporated. Chromatography on silica gel gave 2.1 g (89% of theory) of 6-(3-phenoxyphenyl)-2,3,3-trimethyl-1-hexene.
$n^{20}_{D}$: 1.5443

Example 2

2-(Cyclohexen-1-yl)-2-methyl-1-(4-fluoro-3-phenoxybenzyloxy)propane.

1. 17.62 g (0.2 Mol) isobutyric acid, dissolved in 20 ml THF, was added dropwise to 0.4 Mol lithium diisopropylamide (prepared from diisopropylamine and n-butyllithium, in 120 ml THF at 0° C. The mixture was warmed to room temperature and stirred for one hour at 40-50° C. It was then cooled to 0° C and 19.6 g (0.2 Mol) cyclohexanone, dissolved in 20 ml THF, added dropwise. After stirring for 12 hours at room temperature, the mixture was poured into ice water, made alkaline with caustic soda and shaken with ether. The aqueous phase was acidified with hydrochloric acid and the carboxylic acid extracted with ether. After

washing with water and drying with magnesium sulphate, the extract was evaporated. There remained 21.8 g (58.5% of theory of 2-(1-hydroxycyclohexyl)-2-methylpropionic acid, as a colourless oil. The product was used without further purification.

2. 21.8 g (0.12 Mol) of this hydroxyacid was dissolved in 50 ml ethanol, treated with 3.0 g concentrated sulphuric acid and heated under reflux for 10 hours. The mixture was then poured into ice-water, extracted with ether, the ether phase washed twice with water, dried with magnesium sulphate and evaporated. After chromatography on silica gel there was obtained 13.8 g (58.6% of theory) of ethyl 2-(cyclohexen-1-yl)-2-methylpropionate, as a colourless oil.

3. 1.35 g (35.5 mMol) lithium aluminium hydride was suspended in 40 ml absolute ether. To this was slowly added dropwise 10.0 g (50.9 mMol) of the ester, dissolved in 50 ml ether, whereby the solution came to the boil. After about 2 hours the reaction was complete. Excess hydride was consumed by addition of ethyl acetate, and in turn there was added water and dilute sulphuric acid. The aqueous phase was extracted with ether. The purified ether phase was washed with water, dried and evaporated. There remained 7.1 g (90.5% of theory) 2-(cyclohexen-1-yl)-2-methylpropanol, as a colourless oil, that was used without purification.

4. 0.185 g (6.15 mMol) sodium hydride (80%) was freed from oil by washing twice with toluene and then treated with 10 ml dimethoxyethane. In turn there was added 0.95 g (6.15 mMol) 2-(cyclohexen-1-yl)-2-methylpropanol and 1.69 g (6.0 mMol) 4-fluoro-3-phenoxybenzyl bromide. After stirring for 4 hours at room temperature, the mixture was poured into ice-water, extracted with ether, dried with magnesium sulphate, evaporated and the residue chromatographed on silica gel. There was obtained 1.3 g (61% of theory) of 2-(cyclohexen-1-yl)-2-methyl-1-(4-fluoro-3-phenoxybenzyloxy)propane.
$n^{20}_D$: 1.5464

Example 3

2-Chloro-3,3-dimethyl-4-(4-fluoro-3-phenoxybenzyloxy)-1-butene.

1. 3,3-Dimethyl-4-(4-fluoro-3-phenoxybenzyloxy)butan-2-ol.

20.0 g (66 mMol) 2,2-dimethyl-3-(4-fluoro-3-phenoxybenzyloxy)propanal dissolved in 100 ml ether was added dropwise to a Grignard reagent solution, prepared in ether from 2.23 g (92 mMol) magnesium and 8.7 ml (139 mMol) methyl iodide and the mixture heated under reflux for 2 hours. It was then hydrolysed with dilute hydrochloric acid and the aqueous phase shaken several times with ether. The combined ether phases were washed with water, dried with magnesium sulphate and evaporated. There remained 20.8 g (99% of theory) of an oil that was used without further purification.

2. 3,3-Dimethyl-4-(4-fluoro-3-phenoxybenzyloxy)butan-2-one

20.8 g (66 mMol) of the butanol, dissolved in 130 ml acetone, was treated under ice cooling with Jones reagent (50 mMol $CrO_3$) and the mixture stirred for two hours at room temperature. It was then poured into ice water, extracted several times with ether, the ether phase washed with water, dried, evaporated and the residue purified by silica gel chromatography. After evaporation there remained 16.3 g of a colourless oil (78% of theory).

3. 2-Chloro-3,3-dimethyl-4-(4-fluoro-3-phenoxybenzyloxy)-1-butene.

1.0 g (3.16 mMol) of the ketone was mixed with 1.0 g phosphorus pentachloride and heated for 30 minutes at 70 °C. The mixture was then poured into ice-water, extracted with ether, the ether phase washed with water, dried and evaporated. After chromatography on silica gel there was obtained 0.25 g of the chloro compound (24% of theory).
$n^{20}_D$: 1.5460

In a similar manner, the following compounds were prepared.

| Example No | Process | Compound | $n_D^{20}$ or mp |
|---|---|---|---|
| 4 | a) | 6-(4-fluoro-3-phenoxyphenyl)-2,3,3-trimethyl-1-hexene | 1.5326 |
| 5 | b) | 4-(4-fluoro-3-phenoxybenzyloxy)-2,3,3-trimethyl-1-butene | 1.5330 |
| 6 | d) | 3,3-dimethyl-2-iodo-4-(3-phenoxybenzyloxy)-1-butene | 1.5781 |
| 7 | c) | 2-bromo-3,3-dimethyl-4-(3-phenoxybenzyloxy)-1-butene | 1.5646 |
| 8 | b) | 2-(cyclohexen-1-yl)-2-methyl-1-(3-phenoxybenzyloxy)propane | 1.5540 |
| 9 | b) | 2-(cyclopenten-1-yl)-2-methyl-1-(3-phenoxybenzyloxy)propane | 1.5539 |
| 10 | b) | 2-(cyclopenten-1-yl)-1-(4-fluoro-3-phenoxybenzyloxy)-2-methylpropane | 1.5447 |
| 11 | b) | 2-(cyclohexen-1-yl)-2-methyl-1-(2-methyl-3-phenylbenzyloxy)propane | 1.5644 |
| 12 | b) | 2-(cyclopenten-1-yl)-2-methyl-1-(2-methyl-3-phenylbenzyloxy)propane | 1.5620 |
| 13 | b) | 3,3-dimethyl-4-(3-phenoxybenzyloxy)-2-phenyl-1-butene | 1.5731 |

15

| Example No | Process | Compound | $n_D^{20}$ or mp |
|---|---|---|---|
| 14 | b) | 3,3-dimethyl-4-(4-fluoro-3-phenoxy-benzyloxy)-2-phenyl-1-butene | 1.5635 |
| 15 | b) | 3,3-dimethyl-4-(2-methyl-3-phenyl-benzyloxy)-2-phenyl-1-butene | 38-40°C |
| 16 | b) | 4-(3-phenoxybenzyloxy)-2,3,3-trimethyl-1-butene | 1.5415 |
| 17 | b) | 4-(6-phenoxypyridin-2-ylmethoxy)-2,3,3-trimethyl-1-butene | 1.5405 |
| 18 | a) | 6-(6-phenoxypyridin-2-yl)-2,3,3-trimethyl-1-hexene | 1.5404 |
| 19 | b) | 4-(3-anilinobenzyloxy)-2,3,3-trimethyl-1-butene | |
| 20 | b) | 4-(3-anilino-4-fluorobenzyloxy)-2,3,3-trimethyl-1-butene | 1.5655 |
| 21 | b) | 4-[3-(4-chlorophenoxy)benzyloxy]-2,3,3-trimethyl-1-butene | 1.5491 |
| 22 | b) | 4-[3-(3,4-dichlorophenoxy)-benzyloxy]-2,3,3-trimethyl-1-butene | |
| 23 | b) | 4-[3-(4-t-butylphenoxy)-benzyloxy]-2,3,3-trimethyl-1-butene | |

16

| Example No | Process | Compound | $n_D^{20}$ or mp |
|---|---|---|---|
| 24 | b) | 4-[3-(3-trifluoromethylphenoxy)-benzyloxy]-2,3,3-trimethyl-1-butene | 1.5073 |
| 25 | b) | 3-methylene-5-(3-phenoxy-benzyloxy)-2,4,4-trimethylpentane | 1.5357 |
| 26 | b) | 5-(4-fluoro-3-phenoxybenzyloxy]) 3-methylene-2,4,4-trimethylpentane | 1.5244 |
| 27 | b) | 4,4-dimethyl-3-ethyl-5-(3-phenoxy-benzyloxy)-2-pentene | 1.5371 |
| 28 | b) | 4,4-dimethyl-3-ethyl-5-(4-fluoro-3-phenoxybenzyloxy)-2-pentene | |
| 29 | b) | 5-(3-anilino-4-fluorobenzyloxy)-4,4-dimethyl-3-ethyl-2-pentene | |
| 30 | b) | 4,4-dimethyl-3-ethyl-5-(6-phenoxy-pyridin-2-ylmethoxy)-2-pentene | |
| 31 | a) | 4,4-dimethyl-3-ethyl-7-(6-phenoxy-pyridin-2-yl)-2-heptene | |
| 32 | d) | 2-chloro-3,3-dimethyl-4-(3-phenoxy-benzyloxy)-1-butene | |
| 33 | d) | 2-chloro-3,3-dimethyl-6-(3-phenoxy-phenyl)-1-hexene | |
| 34 | b) | 2-methyl-2-(4-oxocyclohexen-1-yl)-1-(3-phenoxybenzyl)propane | |

17

| Example No | Process | Compound | $n_D^{20}$ or mp |
|---|---|---|---|
| 35 | b) | 1-(4-fluoro-3-phenoxybenzyl)-2-methyl-2-(4-oxocyclohexen-1-yl)propane | |
| 36 | b) | 2-(4-hydroxycyclohexen-1-yl)-2-methyl-1-(3-phenoxybenzyloxy)propane | |
| 37 | b) | 1-(4-fluoro-3-phenoxybenzyloxy)-2-(4-hydroxycyclohexen-1-yl)-2-methylpropane | |
| 38 | b) | 2-(4-ethoxycyclohexen-1-yl)-2-methyl-1-(3-phenoxybenzyloxy)propane | |
| 39 | b) | 2-(4-ethoxycyclohexen-1-yl)-1-(4-fluoro-3-phenoxybenzyloxy)-2-methylpropane | |
| 40 | b) | 2-(4-methoxycyclohexen-1-yl)-2-methyl-1-(3-phenoxybenzyloxy)propane | |
| 41 | b) | 1-(4-fluoro-3-phenoxybenzyloxy)-2-(4-methoxycyclohexen-1-yl)-2-methylpropane | |
| 42 | b) | 2-(1,4-dioxaspiro[4,5]dec-7-en-8-yl)-1-(4-fluoro-3-phenoxybenzyloxy)-2-methylpropane | |
| 43 | b) | 2-(4-azacyclohexen-1-yl)-2-methyl-1-(3-phenoxybenzyloxy)propane | |

| Example No | Process | Compound | $n_D^{20}$ or mp |
|---|---|---|---|
| 44 | b) | 1-(4-fluoro-3-phenoxybenzyloxy)-2-methyl-2-(4-oxacyclohexen-1-yl)propane | 1.5476 |
| 45 | b) | 2-methyl-2-(4-oxacyclohexen-1-yl) 1-(3-phenoxybenzyloxy)propane | 1.5586 |
| 46 | b) | 2-(4-aza-4-methylcyclohexen-1-yl)-2-methyl-1-(3-phenoxybenzyloxy)propane | |
| 47 | b) | 2-(4-aza-4-methylcyclohexen-1-yl)-1-(4-fluoro-3-phenoxybenzyloxy)-2-methyl-propane | |
| 48 | b) | 2-(4-aza-4-isopropylcyclohexen-1-yl)-2-methyl-1-(3-phenoxybenzyloxy)propane | |
| 49 | b) | 2-(4-aza-4-ethoxycarbonylcyclohexen-1-yl)-2-methyl-1-(3-phenoxybenzyloxy)propane | |
| 50 | b) | 2-isopropenyl-2-(3-phenoxybenzyloxy-methyl)cyclopropane | |
| 51 | b) | 2-(4-fluoro-3-phenoxybenzyloxy-methyl)-2-isopropenylcyclopropane | 1.5450 |
| 52 | b) | 2-(3-anilinobenzyloxymethyl)-2-isopropenylcyclopropane | |
| 53 | b) | 2-isopropenyl-2-(6-phenoxypyridin-2-ylmethoxymethyl)cyclopropane | |

19

| Example No | Process | Compound | $n_D^{20}$ or mp |
|---|---|---|---|
| 54 | b) | 2-isopropenyl-2-[3-(3-phenoxyphenyl)-propyl]cyclopropane | |
| 55 | b) | 2-isopropenyl-2-(3-phenoxybenzyloxy-methyl)cyclobutane | |
| 56 | b) | 2-(4-fluoro-3-phenoxybenzyloxy-methyl)-2-isopropenylcyclobutane | |
| 57 | b) | 2-(3-anilino-4-fluorobenzyloxymethyl)-2-isopropenylcyclopropane | |
| 58 | b) | 4-(2,3,4,5,6-pentafluorobenzyloxy)-2,3,3-trimethyl-1-butene | 1.4447 |
| 59 | d) | 2-bromo-3,3-dimethyl-4-(4-fluoro-3-phenoxybenzyloxy)-1-butene | 1.5537 |
| 60 | b) | 3,3-dimethyl-2-iodo-4-(4-fluoro-3-phenoxybenzyloxy)-1-butene | 1.5734 |
| 61 | b) | 3,3-dimethyl-4-(2,3,4,5,6-pentafluoro-benzyloxy)-2-phenyl-1-butene | 1.4960 |
| 62 | b) | 2-(cyclopenten-1-yl)-2-methyl-1-(2,3,4,5,6-pentafluorobenzyloxy)propane | 1.4990 |
| 63 | b) | 3,3-dimethyl-2-iodo-6-(3-phenoxy-phenyl)-1-hexene | 1.5850 |

From the primary leaf of field beans (Phaseolus vulgaris nanus Aschers.), 24 mm diameter discs were cut. Some of these were treated with a 0.1% aqueous preparations of compounds of the invention and these along side untreated discs were placed on filter papers with the underside of the leaves turned upwards. After drying the test pieces, they were each infested with wingless stages of Aphis fabae (approx 100 per leaf piece). The experiment was replicated 3 times. The leaves were kept on wet filter papers for 2 days at 25° C and 16 hours light per day. The percentage mortality was then estimated and the activity calculated using Abbott's method in comparison with the untreated controls.

The compounds of Examples 2, 4, 5, 7, 10, 18, 20, 21, 26, 51, 59, 60 and 64 to 70 showed an activity of 80% or more.

Use Example B

Activity in prophylactic treatment of leaves against brown rice-hoppers (Niliparvata lugens Stal)

Rice seedlings (Oryzae sativa L.) in the two leaf stage (about 10 per polystyrene pot of size 6.5 x 6.5 cm) were either untreated or dipped until dripping wet, with an aqueous preparation containing 0.1% of active material. After drying the sprayed leaves, a transparent cylinder was placed over each pot and through an opening, about 30 brown rice-hoppers (Niliparvata lugens) in the 4-5 stage, anaesthetised with carbon dioxide, were introduced into each pot. After closing the opening with a fine mesh screen, the pots were kept for 2 days at 28° C and 16 hours/day of light in the glasshouse, the amount of dead hoppers was determined. The percentage mortality was then estimated and the activity calculated using Abbott's method in comparison with the untreated controls.

The compounds of Examples 1, 2, 4, 14, 16-18, 20, 21, 24-26, 44, 51, 59, 60, 63-70 and 73 showed an activity of 80% or more.

Use Example C

Activity in the prophylactic treatment of feed against the two spotted mite (Tetranychus urticae Koch)

From the primary leaf of field beans (Phaseolus vulgaris nanus Aschers.) 14 mm diameter discs were cut. Some of these were treated with a 0.1% aqueous preparations of compounds of the invention and these along side untreated discs were placed on filter papers with the underside of the leaves turned upwards. After drying the test pieces, they were each infested with six adult female Tetranychus urticae and maintained for 3 days at 25° C and 16 hours light per day. The experiment was replicated 4 times. Dead and alive adults were then counted and removed. Similarly the number of eggs laid were counted. After a further 7 days, the number of living larvae were counted, the activity calculated using Abbott's method in comparison with the untreated controls.

The compounds of Examples 4, 5, 7, 10, 15, 18, 20, 21, 59, 60, 61, 64, 65 and 68-72 showed 80-100% activity against adults, eggs and larvae.

Use Example D

Activity against eggs/larvae of the corn rootworm (Diabrotica undecimpunctata)

The compounds of the invention were made up as aqueous emulsions at a concentration of 0.1%. Into the soil in polystyrene petri dishes, containing maize seedlings (1 seedling/dish) and ca. 50 eggs of the corn rootworm (Diabrotica undecimpunctata) were pipetted 0.2 ml of these preparations. The closed dishes were left at 25° C under extended daylight conditions for 7 days. The criterion for judging the activity was the death of eggs or newly hatched larvae at the end of the test.

The compounds of Examples 1, 2, 4, 5, 7, 8, 17, 18, 20, 21, 26, 51, 59, 64, 66, 67, 69 and 70 showed 80-100% activity.

Use Example E

Soil insecticide activity against eggs/larvae of the corn rootworm (Diabrotica undecimpunctata)

The compounds of the invention were made up as aqueous preparations with an active ingredient content of 0.0025%. 5 ml of these preparations were pipetted into 300 ml clear plastic beakers, containing 50 $cm^3$ earth and ca. 50 Diabrotica eggs, as well as 2 presoaked maize seeds. The sealed pots were left for 10 days under extended daylight conditions at 25° C. The criterion for judging the activity after 10 days was the larvicidal activity and the root growth of the emerging maize plants in the treated pots in comparison with untreated controls.

It was shown that compounds of Examples 16 and 70 gave 90-100% larvicidal activity as well as allowing good root growth.

Use Example F

Ovicidal activity against eggs of the cotton army worm (Spodoptera littoralis).

The compounds of the invention were made up as aqueous preparations at a concentration of 0.1%. One day old eggs that had been laid on filter paper by fertilised female moths were dipped in the preparations until they were completely wet and then placed in closed petri dishes in the laboratory under extended daylight conditions for four days. The % inhibition of hatching of the eggs in comparison with untreated eggs indicates the level of activity.

The compounds of Examples 4 and 5 showed 80 - 100% activity.

Use Example G

Activity against larvae (L1) of the cotton bollworm (Heliothis viriscens)

Compounds of the invention were made up as aqueous preparations at a concentration of 0.1%. Into these, feed material was dipped for 2 seconds. After drying the feed material was put into into polystyrene petri dishes. After an hour, 10 L1 of the cotton bollworm (Heliothis viriscens) were counted into the dishes. The closed dishes were left for up to 7 days at 25° C under extended daylight conditions. The % mortality of the larvae after two days indicated the level of activity.

The compounds of Examples 4, 5, 8, 10, 20, 59, 62, 63, 64, 67, 69, 70, 71 and 72 showed 80 - 100% activity.

Use Example H

Ovicidal activity against eggs of the cotton bollworm (Heliothis viriscens)

The compounds of the invention were made up as aqueous preparations at a concentration of 0.1%. One day old eggs that had been laid on filter paper by fertilised female moths were dipped in the preparations until they were completely wet and then placed in closed petri dishes under extended daylight conditions for four days. The % inhibition of hatching of the eggs in comparison with untreated eggs indicates the level of activity.

The compound of Example 20 showed 80 - 100% activity.

Use Example I

Activity against Sheep blowfly (Lucilia sericata)

1 ml aliquots of acetone solutions or suspensions, containing test compound at various concentrations, were applied to cotton wool dental rolls (1 cm x 2 cm), contained in glass vials 2 cm diameter x 5 cm long. After drying, the treated materials were then impregnated with 1 ml of nutrient solution, infested with approximately 30 first instar larvae of sheep blow fly (Lucilia sericata), closed by a cotton wool plug and held at 25 °C for 24 hours. For the controls the mortality was <5% whereas compounds of Examples 5 and 16 had an $LC_{50}$ of less than 300ppm.

Use Example J

Activity against house fly (Musca domestica)

Aliquots (0.7 ml) of acetone solutions or suspensions of test compounds at various concentrations were applied to filter papers (9 cm diameter) placed in the bottom of petri dishes (9 cm diameter) closed by glass lids. After evaporation of solvent, the treated surfaces, together with controls treated with acetone alone, were then infested with adult houseflies, (Musca domestica) and held at 22 °C for 24 hours. The percentage mortality of the insects was then recorded. Less than 5% mortality resulted in the controls whereas the $LD_{50}$ of compounds of Examples 5 and 16 was less than 1000 mg/m$^2$.

Test Example K

Tickicidal activity against Boophilus microplus

9 cm diameter filter papers were impregnated with 1 ml aliquots of acetone solutions of test compound at various concentrations. The papers were allowed to dry and then folded into envelopes in which cattle tick larvae, (Boophilus microplus) were enclosed and held at 25 °C and 80% R.H. for 48 hours. The percentage mortality of tick larvae was then recorded and compared with controls.

The controls gave a mortality of less than 5% whereas compounds of Examples 5 and 16 caused 50% mortality at a concentration of 100 ppm or less.

**Claims**

1. Substituted alkenes of general formula I

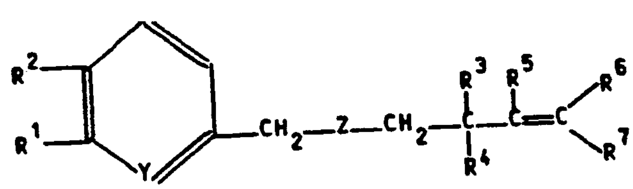

(I),

in which

$R^1$ is phenyl, phenoxy or anilino, which can be substituted by halogen, $C_{1-4}$-alkyl or $C_{1-4}$-alkoxy;

$R^2$ is hydrogen or fluorine;

$R^3$ and $R^4$, independently of each other, are $C_{1-4}$-alkyl or together form a 3-6 membered ring;

$R^5$ is $C_{1-6}$-alkyl, $C_{1-6}$-haloalkyl, phenyl or halogen;

$R^6$ is hydrogen, halogen, $C_{1-6}$-alkyl, $C_{1-6}$-haloalkyl, $C_{2-6}$-alkenyl or phenyl; or

$R^5$ and $R^6$ together form a ring, which can be substituted by halogen or alkyl and can contain a further double bond and/or an O, N or S atom;

$R^7$ is hydrogen, halogen or $C_{1-4}$-alkyl

Y is CH, $CCH_3$ or N; and

Z is O, S or $CH_2$.

2. An insecticidal composition which comprises a compound claimed in claim 1, in admixture with an agriculturally acceptable diluent or carrier.

3. A method of combating insects, which comprises applying to the insect or its locus an effective amount of a compound claimed in claim 1.

European Patent Office

# EUROPEAN SEARCH REPORT

Application Number

EP 90 25 0006

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | CHEMICAL ABSTRACTS, vol. 102, no. 1, 7th January 1985, page 535, abstract no. 5911g, Columbus, Ohio, US; & JP-A-59 116 243 (MITSUI TOATSU CHEMICALS, INC.) 05-07-1984<br>* Page 41755CS, Naphthalene, 1,2-dihydro-3-[1-[[(3-phenoxy-phenyl)methoxy]methyl]cyclopropyl]- * & CHEMICAL ABSTRACTS ELEVENTH COLLECTIVE INDEX, vol. 96-105, 1982-1986, Chemical Substances, Molybdenum alloy-Nichilon<br>--- | 1-3 | C 07 C 43/29<br>A 01 N 31/08<br>A 01 N 31/04<br>A 01 N 33/08<br>A 01 N 43/02<br>A 01 N 43/40<br>C 07 C 43/285<br>C 07 C 43/295<br>C 07 C 43/164<br>C 07 C 49/753<br>C 07 C 217/76<br>C 07 D 213/643<br>C 07 D 295/00<br>C 07 D 309/22<br>C 07 D 317/72 |
| D,A | EP-A-0 227 369 (ICI)<br>* Claims; tables I,II,III *<br>--- | 1-3 | |
| A | FR-A-2 342 953 (SHELL INTERNATIONALE RESEARCH)<br>* Claims; example 13 *<br>----- | 1-3 | |

TECHNICAL FIELDS SEARCHED (Int. Cl.5)

C 07 C 43/00
C 07 C 49/00
C 07 D 217/00
C 07 D 213/00
C 07 D 295/00
C 07 D 309/00
C 07 D 317/00

The present search report has been drawn up for all claims

| Place of search | Date of completion of the search | Examiner |
|---|---|---|
| THE HAGUE | 20-04-1990 | WRIGHT M.W. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P0401)